# EUROPÄISCHE PATENTSCHRIFT

(11) **EP 2 301 488 B1**
(45) Veröffentlichungstag und Bekanntmachung des Hinweises auf die Patenterteilung: **17.04.2013**
(21) Anmeldenummer: 10405177.6
(22) Anmeldetag: 21.09.2010
(51) Int. Cl.: A61F 5/01

(54) **Ruhigstellschiene**
Immobilizing rail
Rail de mise au repos

(30) Priorität: 24.09.2009 CH 14742009
(43) Veröffentlichungstag der Anmeldung: 30.03.2011
(73) Patentinhaber: MIC & Partners GmbH, 8052 Zürich (CH)
(72) Erfinder: Natsis, Michael, 8052 Zürich (CH)
(74) Vertreter: Detken, Andreas

(56) Entgegenhaltungen:
- WO-A1-2005/087150
- DE-U1- 20 007 196
- US-A- 5 899 872

## Beschreibung

Die Erfindung betrifft eine Ruhigstellschiene nach dem Oberbegriff des Anspruchs 1.

Schienen zur Ruhigstellung eines Fusses nach einer Verletzung, insbesondere einem Knochenbruch, sind seit langem allgemein bekannt. Zum Stand der Technik wird hier beispielsweise auf die US 5,899,872, US 4,919,118, US 5,399,152, EP 0 355 930 und DE 101 63 706 B4 verwiesen. Diese eignen sich jedoch in der Regel lediglich in einer späteren Phase der Heilung, in welcher der Fuss belastbar ist. Bis es soweit ist, muss der Fuss mit einem Gipsprovisorium versehen sein. Während dieser Phase muss der Fuss abschwellen, bis schliesslich ein üblicher Gipsverband bzw. eine der oben genannten Schalen angelegt werden kann. Das Herstellen eines Gipsprovisoriums ist vergleichsweise aufwendig und erfordert auch einen längeren Einsatz von Fachpersonal.

Die DE-U-200 07 196, der als nächster Stand der Technik augesehen wird, offenbart eine Orthese zur Behandlung von Erkrankungen und Verletzungen am oberen und unteren Sprunggelenk. Sie besitzt eine Schiene mit einem Sohlenteil und an der Schiene angebrachten Bändern. Die Orthese eignet sich nicht als Ersatz für einen Gipsverband.

Die WO-A-2005/087150 offenbart eine Vorrichtung, die entweder als Knöchelstütze oder Kniestütze vorgesehen ist und die an die Form dieser Teile anpassbare Elemente aufweist. Die Vorrichtung ist als Stütze vorgesehen, die eine Sportausübung ermöglichen soll. Als Ersatz für ein Gipsprovisorium ist diese Vorrichtung ebenfalls nicht geeignet.

Der Erfindung liegt die Aufgabe zugrunde, eine Ruhigstellschiene der genannten Art zu schaffen, die sich insbesondere als Ersatz für ein Gipsprovisorium eignet.

Die Aufgabe ist gemäss Anspruch 1 gelöst. Die erfindungsgemässe Ruhigstellschiene eignet sich insbesondere dann, wenn ein Fuss mit einem Winkel von 90° ruhiggestellt werden soll, wobei der Fuss nicht belastet wird. Mit dem Einstellelement ist es möglich, die Schiene exakt an den Fuss anzupassen, so dass in der Ruhigstellposition Druckstellen vermieden werden können. Die Ruhigstellschiene ist an sehr unterschiedlich grosse Füsse anpassbar. Wesentlich ist, dass beim Abschwellen des Fusses während der postoperativen Phase kein Nachgipsen eines Gipsprovisoriums erforderlich ist. Dies erspart erheblich weiteren Materialeinsatz und kostspielige Einsatzzeiten der behandelnden Personen. Die Ruhigstellschiene kann sehr einfach beispielsweise mit einem elastischen Band fixiert werden. Ein weiterer Vorteil wird auch darin gesehen, dass nach einer Operation insbesondere in einem Notfall keine Wartezeiten erforderlich sind, wie dies bisher bei einem Gipsverband erforderlich war. Die erfindungsgemässe Ruhigstellschiene kann zudem mit einem sehr geringen Gewicht hergestellt werden. Beim Abschwellen des Fusses ist kein Wechsel erforderlich. Zudem kann die Ruhigstellschiene röntgendurchlässig hergestellt werden.

Nach einer Weiterbildung der Erfindung ist vorgesehen, dass das Einstellelement eine Platte ist, welche mit der Fussschale bzw. der Unterschenkelschale verbunden ist. Eine solche Platte kann im Wesentlichen beliebig verformt und damit an den Fuss bzw. an den Unterschenkel genau angepasst werden. Im Fall der Fussschale ist vorgesehen, dass das Einstellelement sich von einem über der Ferse angeordneten Bereich seitlich nach unten bis zum Aussenknöchel und Innenknöchel erstreckt. Vorzugsweise befindet sich das Einstellelement über einer Ausnehmung für die Ferse und die Achillessehne. Das Einstellelement kann vorteilhafterweise als einstückige Platte hergestellt werden, die im Wesentlichen U-förmig ist und sich seitlich nach unten erstreckt.

Das Einstellelement ist gemäss einer Weiterbildung der Erfindung aus Metall hergestellt. Das Metall ist vorzugsweise und beispielsweise Aluminium. Die Platte kann an der Fussschale bzw. an der Unterschenkelschale aussenseitig mit geeigneten Befestigungselementen, beispielsweise Nieten befestigt sein. Das Einstellelement kann aber auch eingebettet und beispielsweise umspritzt sein. Ein solches Einstellelement ist plastisch, beispielsweise mit einem geeigneten Werkzeug verformbar, dennoch aber vergleichsweise stabil. Dadurch kann die Form der Fussschale bzw. der Unterschenkelschale fixiert werden. Diese Schalen sind elastisch und beispielsweise aus Kunststoff hergestellt.

Die Fussschale und die Unterschenkelschale werden vorzugsweise durch eine einstückige Schale gebildet. Diese kann beispielsweise im Spritzgussverfahren aus einem geeigneten Kunststoff hergestellt werden. Da die Fussschale und die Unterschenkelschale vorne offen sind, kann sie sehr einfach von der Rückseite her an den Fuss bzw. an den Unterschenkel angelegt werden. Druckstellen können dann besonders wirksam vermieden werden, wenn die Fussschale und die Unterschenkelschale innenseitig mit einem Polster versehen sind. Der Fuss und der Unterschenkel werden vor dem Anlegen der Ruhigstellschiene vorzugsweise bandagiert.

Nach einer Weiterbildung der Erfindung ist vorgesehen, dass die Ruhigstellschiene im Wesentlichen spiegelsymmetrisch ausgebildet ist. Sie kann dann wahlweise an ein linkes oder rechtes Bein angelegt werden. Die entsprechende Form ergibt sich durch die Verformung des wenigstens einen Einstellelementes.

Vorzugsweise sind wenigstens zwei Einstellelement vorgesehen. Das eine Einstellelement ist an der Fussschale und das andere an der Unterschenkelschale angeordnet. Beide Einstellelemente können unabhängig voneinander verformt werden. Sie können aus dem gleichen oder aus verschiedenen Materialien und insbesondere verschiedenen oder gleichen Metallen hergestellt sein.

Weitere vorteilhafte Merkmale ergeben sich aus den abhängigen Patentansprüchen, der nachfolgenden Beschreibung sowie der Zeichnung.

Ein Ausführungsbeispiel der Erfindung wird nachfolgend anhand der Zeichnung näher erläutert. Es zeigen:
- Fig. 1: eine räumliche Ansicht einer erfindungsgemässen Ruhigstellschiene,
- Fig. 2: eine weitere Ansicht der Ruhigstellschiene nach Fig. 1,
- Fig. 3: eine weitere Ansicht der erfindungsgemässen Ruhigstellschiene angelegt an einen Fuss,
- Fig. 4: eine weitere Ansicht eines Beines mit einer angelegten erfindungsgemässen Ruhigstellschiene und
- Fig. 5: eine weitere Ansicht eines Fusses mit einer Ruhigstellschiene und ein Fuss ohne eine solche Schiene.

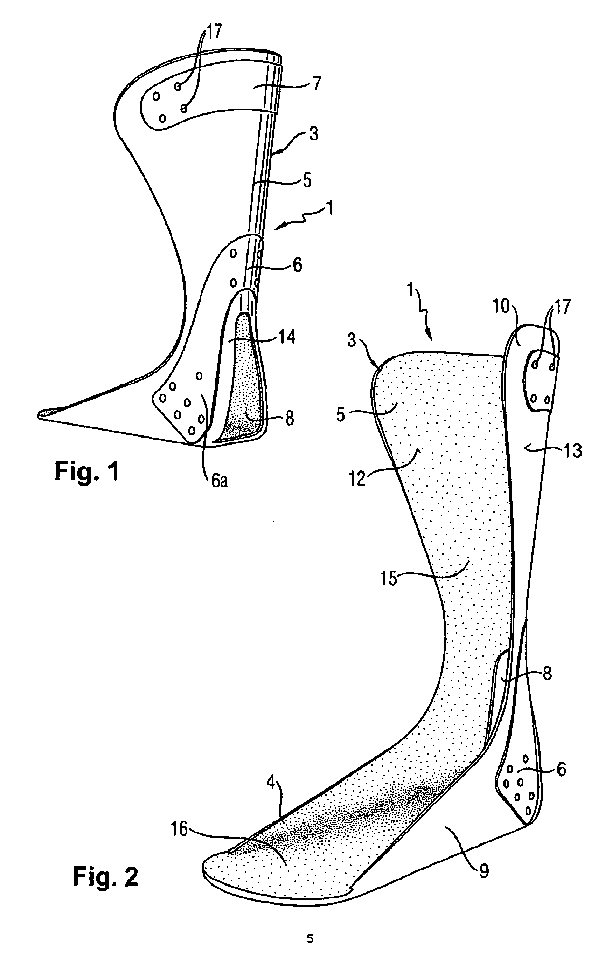

Die erfindungsgemässe Ruhigstellschiene 1 besitzt gemäss den Fig. 1 und 2 eine Schale 3 die aus einem flexiblen Kunststoff hergestellt ist und an einer Innenseite 12 eine Polsterung 15 aufweist. Diese Polsterung 15 besteht beispielsweise aus einem Schaumstoff und erstreckt sich über die gesamte Innenseite 12. Die Schale 3 besteht aus einer Fussschale 4 und einer Unterschenkelschale 5, die an zwei Übergangsbereichen 14 miteinander verbunden sind. Die Fussschale 4 besitzt eine Sohle 16 und zwei gegenüberliegende Seitenwandungen 9. An der Rückseite im Bereich der Ferse und der Achillsehne besitzt zudem die Fussschale 4 eine Öffnung 8. Diese Öffnung erstreckt sich nach oben bis in den Bereich der beiden oben genannten Übergangsbereiche 14. An einer Vorderseite sind die Fussschale 4 und die Unterschenkelschale 5 offen. Die Oberschenkelschale 5 ist zudem noch oben offen. Die Ruhigstellschiene 1 kann somit von der Rückseite an den Fuss bzw. an das Bein angelegt werden.

An einer Aussenseite 13 der Schale 3 sind ein Einstellelement 6 und ein Einstellelement 7 befestigt. Die Befestigung erfolgt beispielsweise mittels Nieten 17 oder anderen geeigneten Befestigungsmitteln. Denkbar ist auch ein Verkleben oder Verschweissen oder auch ein Einlaminieren. Die beiden Einstellelemente 6 und 7 sind vorzugsweise Metallplatten, die beispielsweise mit einem geeigneten Instrument verformbar sind. Sie sind dennoch vergleichsweise formstabil, so dass sie die Form der flexiblen Schale 3 fixieren. Die Einstellelemente 6 und 7 sind beispielsweise aus Aluminiumblech hergestellt. Das Einstellelement 6 ist wie das Einstellelement 7 im Wesentlichen U-förmig ausgebildet und erstreckt sich gemäss der Fig. 1 von einem über der Öffnung 8 angeordneten Bereich seitlich nach unten. In der Fig. 1 ist der linke Bereich 6a gezeigt, der sich seitlich über den linken Knöchel erstreckt. Spiegelsymmetrisch gegenüber liegt ein weiterer Bereich, welcher sich über dem rechten Knöchel befindet. Das Einstellelement 7 ist am oberen Ende der Unterschenkelschale 5 angeordnet und erstreckt sich etwa halbkreisförmig um die Wade.

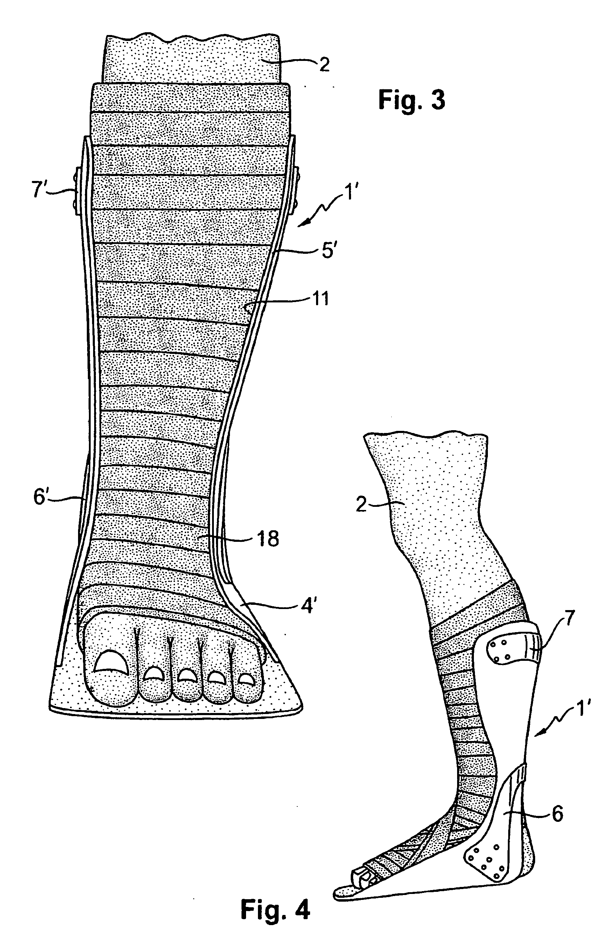

Vor der Verformung der Einstellelemente 6 und 7 ist die Ruhigstellschiene 1 spiegelsymmetrisch. Sie kann wahlweise an den linken oder an den rechten Fuss angelegt werden. Durch Verformen der Einstellelemente 6 und 7 wird dann die Ruhigstellschiene 1 an den entsprechenden Fuss angepasst, so dass dann die Spiegelsymmetrie verloren geht. Dies ist beispielsweise in Fig. 3 gezeigt. Die beiden Einstellelemente 6' und 7' sind hier nun verformt und an das Bein 2 bzw. an den Fuss 18 angepasst. Die Unterschenkelschale 5 umgibt halbkreisförmig die Wade und entsprechend ist die frontseitige Öffnung 11 an den Unterschenkel angepasst. Der Fuss 18 ist wie ersichtlich von der Fussschale 4' umfasst. Die Fussschale 4' ist wie die Unterschenkelschale 5' an das entsprechende Glied angepasst. Dies ist ebenfalls aus Fig. 4 ersichtlich.

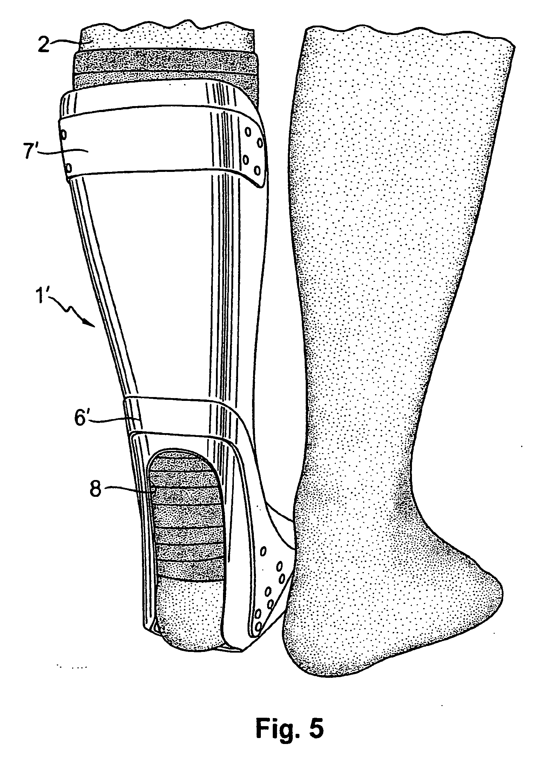

Die Fig. 5 zeigt ebenfalls links ein Bein 2, an welches die angepasste Ruhigstellschiene 1' angelegt ist. Die Ferse und der Bereich der Achillessehne sind aufgrund der Aussparung bzw. Öffnung 8 frei und damit nicht belastet. Beim Abschwellen des Fusses 18 kann die Ruhigstellschiene 1' entsprechend durch Verformen der Einstellelemente 6' und 7' weiter angepasst werden. Die Ruhigstellschiene 1 kann zudem sehr einfach abgenommen werden.

### BEZUGSZEICHENLISTE

| | |
|---|---|
| 1 | Ruhigstellschiene |
| 2 | Bein |
| 3 | Schale |
| 4 | Fussschale |
| 5 | Unterschenkelschale |
| 6 | Einstellelement (Fuss) |
| 7 | Einstellelement (Wade) |
| 8 | Öffnung |
| 9 | Seitenwandung |
| 10 | Wandung |
| 11 | Öffnung |
| 12 | Innenseite |
| 13 | Aussenseite |
| 14 | Übergangsbereich |
| 15 | Polsterung |
| 16 | Sohle |
| 17 | Befestigungselement |
| 18 | Fuss |

## Patentansprüche

1. Ruhigstellschiene zur fixierenden Aufnahme eines Fusses (2), mit einer Fussschale (4) und einer Unterschenkelschale (5), die miteinander verbunden und jeweils an einer Vorderseite offen sind und die an einen Fuss bzw. einen Unterschenkel anlegbar sind, **dadurch gekennzeichnet, dass** die Fussschale (4) und die Unterschenkelschale (5) elastisch ausgebildet sind und dass wenigstens ein Einstellelement (6, 7) vorgesehen ist, das zur Anpassung und Stabilisierung der Form der Fussschale (4) verformbar ist, wobei die Fussschale (4) eine Sohle (16) und zwei gegenüberliegende Seitenwandungen (9) aufweist.

2. Schiene nach Anspruch 1, **dadurch gekennzeichnet, dass** das Einstellelement (6, 7) eine Platte ist, welche mit der Fussschale (4) oder der Unterschenkelschale (5) verbunden ist.

3. Schiene nach Anspruch 1 oder 2, **dadurch gekennzeichnet, dass** die Fussschale (4) ein Einstellelement (6) aufweist, das sich von einem über der Ferse angeordneten Bereich seitlich nach unten bis zum Aussenknöchel und Innenknöchel erstreckt.

4. Schiene nach einem der Ansprüche 1 bis 3, **dadurch gekennzeichnet, dass** die Fussschale (4) rückseitig eine Ausnehmung (8) für die Ferse und die Achillessehne aufweist.

5. Schiene nach Anspruch 3 oder 4, **dadurch gekennzeichnet, dass** das Einstellelement (6) der Fussschale (4) im Wesentlichen U-förmig ausgebildet ist.

6. Schiene nach Anspruch 5, **dadurch gekennzeichnet, dass** das Einstellelement (6) der Fussschale (4) eine einstückige plastisch verformbare Platte ist.

7. Schiene nach einem der Ansprüche 1 bis 6, **dadurch gekennzeichnet, dass** die Unterschenkelschale (5) ein Einstellelement (7) aufweist, das sich rückseitig etwa halbkreisförmig um die Wade erstreckt.

8. Schiene nach Anspruch 7, **dadurch gekennzeichnet, dass** das Einstellelement (7) der Unterschenkelschale (5) an einem oberen Ende der Unterschenkelschale (5) angebracht ist und sich im Wesentlichen horizontal erstreckt.

9. Schiene nach Anspruch 7 oder 8, **dadurch gekennzeichnet, dass** das Einstellelement (7) der Unterschenkelschale (5) im Wesentlichen U-förmig ausgebildet ist.

10. Schiene nach einem der Ansprüche 1 bis 9, **dadurch gekennzeichnet, dass** das Einstellelement (6, 7) aus Metall hergestellt ist.

11. Schiene nach Anspruch 10, **dadurch gekennzeichnet, dass** das Metall Aluminium ist.

12. Schiene nach einem der Ansprüche 1 bis 11, **dadurch gekennzeichnet, dass** die Fussschale (4) und die Unterschenkelschale (5) Bereiche einer einstückig hergestellten Schale (3) sind.

13. Schiene nach einem der Ansprüche 1 bis 12, **dadurch gekennzeichnet, dass** die Fussschale (4) und die Unterschenkelschale (5) an einer Innenseite (12) ein Polster (15) aufweisen.

14. Schiene nach einem der Ansprüche 1 bis 13, **dadurch gekennzeichnet, dass** sie im Wesentlichen spiegelsymmetrisch ist, so dass sie wahlweise an ein linkes oder rechtes Bein anlegbar ist.

## Claims

1. An immobilizing splint to receive a foot (2) in a fixing manner, with a foot shell (4) and a lower leg shell (5), which are connected with one another and are respectively open at a front side and which are able to be applied onto a foot or respectively a lower leg, **characterized in that** the foot shell (4) and the lower leg shell (5) are constructed so as to be elastic and that at least one adjusting element (6, 7) is provided, which is deformable for adapting and stabilizing the shape of the foot shell (4), wherein the foot shell (4) has a sole (16) and two opposite side walls (9).

2. The splint according to Claim 1, **characterized in that** the adjusting element (6, 7) is a plate which is connected with the foot shell (4) or with the lower leg shell (5).

3. The splint according to Claim 1 or 2, **characterized in that** the foot shell (4) has an adjusting element (6) which extends from a region arranged over the heel laterally downwards to the lateral malleolus and medial malleolus.

4. The splint according to one of Claims 1 to 3, **characterized in that** the foot shell (4) has on the rear side a recess (8) for the heel and the Achilles tendon.

5. The splint according to Claim 3 or 4, **characterized in that** the adjustment element (6) of the foot shell (4) is constructed substantially in a U-shape.

6. The splint according to Claim 5, **characterized in that** the adjusting element (6) of the foot shell (4) is a single-piece plastically deformable plate.

7. The splint according to one of Claims 1 to 6, **characterized in that** the lower leg shell (5) has an adjusting element (7), which extends on the rear side approximately in a semicircular shape around the calf.

8. The splint according to Claim 7, **characterized in that** the adjusting element (7) of the lower leg shell (5) is mounted at an upper end of the lower leg shell (5) and extends substantially horizontally.

9. The splint according to Claim 7 or 8, **characterized in that** the adjusting element (7) of the lower leg shell (5) is constructed substantially in a U-shape.

10. The splint according to one of Claims 1 to 9, **characterized in that** the adjusting element (6, 7) is produced from metal.

11. The splint according to Claim 10, **characterized in that** the metal is aluminium.

12. The splint according to one of Claims 1 to 11, **characterized in that** the foot shell (4) and the lower leg shell (5) are regions of a shell (3) which is produced in one piece.

13. The splint according to one of Claims 1 to 12, **characterized in that** the foot shell (4) and the lower leg shell (5) have a pad (15) on an inner side (12).

14. The splint according to one of Claims 1 to 13, **characterized in that** it is substantially mirror-symmetrical, so that it is able to be applied selectively to a left or a right leg.

## Revendications

1. Rail de mise au repos pour recevoir un pied (2) de manière à l'immobiliser, comprenant une coque pour le pied (4) et une coque pour le bas de jambe (5) qui sont reliées ensemble et respectivement ouvertes à l'avant, et que l'on peut appliquer à un pied ou un bas de jambe, **caractérisé en ce que** la coque pour le pied (4) et la coque pour le bas de jambe (5) sont réalisées de manière élastique et **en ce qu'**au moins un élément de réglage (6, 7) est prévu qui peut se déformer pour adapter et stabiliser la forme de la coque pour le pied (4), dans lequel la coque pour le pied (4) présente une semelle (16) et deux parois latérales opposées (9).

2. Rail selon la revendication 1, **caractérisé en ce que** l'élément de réglage (6, 7) est une plaque reliée à la coque pour le pied (4) ou à la coque pour le bas de jambe (5).

3. Rail selon la revendication 1 ou 2, **caractérisé en ce que** la coque pour le pied (4) présente un élément de réglage (6) qui s'étend à partir d'une zone disposée au-dessus du talon latéralement vers le bas jusqu'à la cheville extérieure et la cheville intérieure.

4. Rail selon l'une quelconque des revendications 1 à 3, **caractérisé en ce que** la coque pour le pied (4) présente à l'arrière un creux (8) pour le talon et le tendon d'Achille.

5. Rail selon la revendication 3 ou 4, **caractérisé en ce que** l'élément de réglage (6) de la coque pour le pied (4) est réalisé substantiellement en forme de U.

6. Rail selon la revendication 5, **caractérisé en ce que** l'élément de réglage (6) de la coque pour le pied (4) est une plaque intégrale à déformation plastique.

7. Rail selon l'une quelconque des revendications 1 à 6, **caractérisé en ce que** la coque pour le bas de jambe (5) présente un élément de réglage (7) qui s'étend à l'arrière autour du mollet approximativement de manière semi-circulaire.

8. Rail selon la revendication 7, **caractérisé en ce que** l'élément de réglage (7) de la coque pour le bas de jambe (5) est attaché à une extrémité supérieure de la coque pour le bas de jambe (5) et s'étend de manière substantiellement horizontale.

9. Rail selon la revendication 7 ou 8, **caractérisé en ce que** l'élément de réglage (7) de la coque pour le bas de jambe (5) est réalisé substantiellement en forme de U.

10. Rail selon l'une quelconque des revendications 1 à 9, **caractérisé en ce que** l'élément de réglage (6, 7) est fabriqué en métal.

11. Rail selon la revendication 10, **caractérisé en ce que** le métal est de l'aluminium.

12. Rail selon l'une quelconque des revendications 1 à 11, **caractérisé en ce que** la coque pour le pied (4) et la coque pour le bas de jambe (5) sont des zones d'une coque (3) fabriquée d'une seule pièce.

13. Rail selon l'une quelconque des revendications 1 à 12, **caractérisé en ce que** la coque pour le pied (4) et la coque pour le bas de jambe (5) présentent un matelassage (15) sur une face intérieure (12).

14. Rail selon l'une quelconque des revendications 1 à 13, **caractérisé en ce qu'**il se présente substantiellement en symétrie axiale de sorte qu'il peut être appliqué au choix à la jambe gauche ou droite.
